# EUROPEAN PATENT SPECIFICATION

(11) **EP 3 621 614 B1**
(45) Date of publication and mention of the grant of the patent: **25.06.2025**
(21) Application number: 18727965.8
(22) Date of filing: 11.05.2018
(51) Int. Cl.: A61K 31/4436, A61K 9/00, A61K 47/14, A61K 47/32, A61K 9/107, A61P 17/10, A61K 9/06, A61K 47/44

(54) **LOTION COMPRISING TAZAROTENE FOR TOPICAL USE IN THE TREATMENT OF ACNE**
TAZAROTENE ENTHALTENDE LOTION ZUR TOPISCHEN ANWENDUNG BEI DER BEHANDLUNG VON AKNE
LOTION COMPRENANT DU TAZAROTÈNE POUR UTILISATION TOPIQUE DANS LE TRAITEMENT DE L'ACNÉ

(30) Priority: 12.05.2017 US 201762505421 P
(43) Date of publication of application: 18.03.2020
(73) Proprietor: Bausch Health Ireland Limited, Dublin 24 (IE)
(72) Inventor: ANGEL, Arturo, Santa Rosa California 95409 (US); PILLAI, Radhakrishnan, Santa Rosa, California 95409 (US)
(74) Representative: Maiwald GmbH
(86) International application number: PCT/US2018/032359
(87) International publication number: WO 2018/209262

(56) References cited:
- WO-A1-2016/205001
- CN-A- 1 528 328
- SHALITA A R ET AL: "Tazarotene gel is safe and effective in the treatment of acne vulgaris: a multicenter, double-blind, vehicle-controlled study.", CUTIS JUN 1999, vol. 63, no. 6, June 1999 (1999-06-01), pages 349 - 354, ISSN: 0011-4162
- KIRCIK LEON H: "Tretinoin microsphere gel pump 0.04% versus tazarotene cream 0.05% in the treatment of mild-to-moderate facial acne vulgaris.", JOURNAL OF DRUGS IN DERMATOLOGY : JDD JUL 2009, vol. 8, no. 7, July 2009 (2009-07-01), pages 650 - 654, ISSN: 1545-9616
- ANONYMOUS: "Tazorac package leaflet", 1 July 2017 (2017-07-01), XP055960856, Retrieved from the Internet <URL:https://media.allergan.com/actavis/actavis/media/allergan-pdf-documents/product-prescribing/2017-TAZORAC-Cream-USPI-FINAL_8-7-17.pdf> [retrieved on 20220914]
- ANONYMOUS: "FDA's Summary Basis of Approval of Tazorac", 11 March 2004 (2004-03-11), XP055960867, Retrieved from the Internet <URL:https://www.accessdata.fda.gov/drugsatfda_docs/nda/2000/21-184_Tazorac_Medr_P2.pdf> [retrieved on 20220914]
- DATABASE MEDLINE [online] US NATIONAL LIBRARY OF MEDICINE (NLM), BETHESDA, MD, US; June 1999 (1999-06-01), SHALITA A R ET AL: "Tazarotene gel is safe and effective in the treatment of acne vulgaris: a multicenter, double-blind, vehicle-controlled study.", Database accession no. NLM10388959
- SHALITA A R ET AL: "Tazarotene gel is safe and effective in the treatment of acne vulgaris: a multicenter, double-blind, vehicle-controlled study.", CUTIS JUN 1999, vol. 63, no. 6, June 1999 (1999-06-01), pages 349 - 354, ISSN: 0011-4162
- DATABASE MEDLINE [online] US NATIONAL LIBRARY OF MEDICINE (NLM), BETHESDA, MD, US; July 2009 (2009-07-01), KIRCIK LEON H: "Tretinoin microsphere gel pump 0.04% versus tazarotene cream 0.05% in the treatment of mild-to-moderate facial acne vulgaris.", Database accession no. NLM19588641
- KIRCIK LEON H: "Tretinoin microsphere gel pump 0.04% versus tazarotene cream 0.05% in the treatment of mild-to-moderate facial acne vulgaris.", JOURNAL OF DRUGS IN DERMATOLOGY : JDD JUL 2009, vol. 8, no. 7, July 2009 (2009-07-01), pages 650 - 654, ISSN: 1545-9616
- TANGHETTI EMIL A. ET AL: "A Phase 2, Multicenter, Double-Blind, Randomized, Vehicle-Controlled Clinical Study to Compare the Safety and Efficacy of a Novel Tazarotene 0.045% Lotion and Tazarotene 0.1% Cream in the Treatment of Moderate-to-Severe Acne Vulgaris", JOURNAL OF DRUGS IN DERMATOLOGY, vol. 18, no. 6, 1 June 2019 (2019-06-01), US, pages 542, XP093192660, ISSN: 1545-9616
- BHATIA NEAL ET AL: "Novel Polymeric Tazarotene 0.045% Lotion for Moderate-to-Severe Acne: Pooled Phase 3 Analysis by Race/Ethnicity", JOURNAL OF DRUGS IN DERMATOLOGY, vol. 19, no. 7, 1 July 2020 (2020-07-01), US, pages 727 - 734, XP093192728, ISSN: 1545-9616, DOI: 10.36849/JDD.2020.5125
- KIRCIK LEON H: "Tretinoin microsphere gel pump 0.04% versus tazarotene cream 0.05% in the treatment of mild-to-moderate facial acne vulgaris.", JOURNAL OF DRUGS IN DERMATOLOGY : JDD JUL 2009, vol. 8, no. 7, July 2009 (2009-07-01), pages 650 - 654, XP009506864, ISSN: 1545-9616
- SHALITA A R ET AL: "Tazarotene gel is safe and effective in the treatment of acne vulgaris: a multicenter, double-blind, vehicle-controlled study.", CUTIS JUN 1999, vol. 63, no. 6, June 1999 (1999-06-01), pages 349 - 354, XP009506866, ISSN: 0011-4162

## Description

### BACKGROUND OF THE INVENTION

The present invention relates to topical compositions for use in the treatment of acne. In particular, this invention relates to topical pharmaceutical compositions comprising tazarotene or a pharmaceutically acceptable tazarotenic acid salt, in an oil-in-water emulsion vehicle, for treating skin diseases.

The retinoid tazarotene has been commercially available and has been used to treat acne and psoriasis topically. However, tazarotene may cause significant local skin irritation, especially early in the first through fourth weeks of therapy, thus limiting its use for a prolonged period because many patients stop treatment due to skin irritation. The need exists for more effective and safer topical medicaments with reduced adverse effects for the management of acne, psoriasis and other skin diseases.

WO 2016/205001 A1 is concerned with topical pharmaceutical compositions comprising a combination of a corticosteroid and a retinoid; and methods for treating psoriasis with same. CN1528328 A relates to a compound medicine for external application for acne. Its composition contains the following two active components: (wt%) 0.01-2% of tazarotene and 0.25-5% of antimicrobial. Said compound medicine can be made into emulsifiable paste or gel or liniment or film preparation.

The article *"*Tretinoin microsphere gel pump 0.04% versus tazarotene cream 0.05% in the treatment of mild-to-moderate facial acne vulgaris" of L.H. Kircik (Journal of Drugs in Dermatology 2009, 8, 650-654) describes a 12 weeks, single-center, investigator-blinded, randomized, parallel-design study assessing the safety and efficacy of tretinoin microsphere gel 0.04% delivered by pump to tazarotene cream 0.05% in the treatment of mild-to-moderate facial acne vulgaris.

The article *"*Tazarotene gel is safe and effective in the treatment of acne vulgaris: a multicenter, double-blind, vehicle-controlled study" by A. R. Shalita et al. (Cutis 1999, 63, 349-354) describes a study evaluating the safety and efficacy of topical tazarotene 0.1% and 0.05% gels in comparison to vehicle gel, applied once daily for 12 weeks, in the treatment of mild-to-moderate facial acne vulgaris.

### SUMMARY OF THE INVENTION

In general, the present invention provides topical compositions for use in methods for treating acne.

This invention provides a topical pharmaceutical composition for use in the treatment of acne, comprising tazarotene or a pharmaceutically acceptable tazarotenic acid salt present in the composition at 0.01 to 0.045 percent by weight of the composition; and a dermatologically acceptable oil-in-water emulsion vehicle,the topical pharmaceutical composition containing the tazarotene or the tazarotenic acid salt as a sole active pharmaceutical ingredient, wherein the topical pharmaceutical composition is a lotion.

According to one aspect, the tazarotene or tazarotenic acid salt may be dissolved in a liquid oil component of the emulsion.

According to various aspects, the oil phase of the emulsion may comprise a liquid oil component comprising a DCAE, a MCAE, or combinations thereof. In other aspects, the oil phase includes diethyl sebacate as the DCAE.

According to other aspects, the aqueous phase of the emulsion may comprise water and a carbomer homopolymer.

According to another aspect, the topical pharmaceutical composition may comprise: an aqueous phase comprising water, a carbomer homopolymer, and a polymeric emulsifier; an oil phase comprising at least one member selected from the group consisting of a dicarboxylic acid ester and a mineral oil, and the tazarotene or the tazarotenic acid salt. According to other aspects, the aqueous phase may further comprise a humectant or a preservative, such as at least one member selected from the group consisting of methylparaben, propylparaben and sorbitol.

According to another aspect, the topical pharmaceutical composition may have a pH of about 4 to about 6.

According to this invention, the step of applying may be carried out once per day for at least eight weeks. The applying is carried out once per day for at least twelve weeks.

According to other aspects, the composition may be applied is to an affected area of a body of a subject suffering from acne vulgaris in order to treat that condition.

Other features and advantages of the present invention will become apparent from the accompanying drawings and the following detailed description and claims.

### BRIEF DESCRIPTION OF THE FIGURES

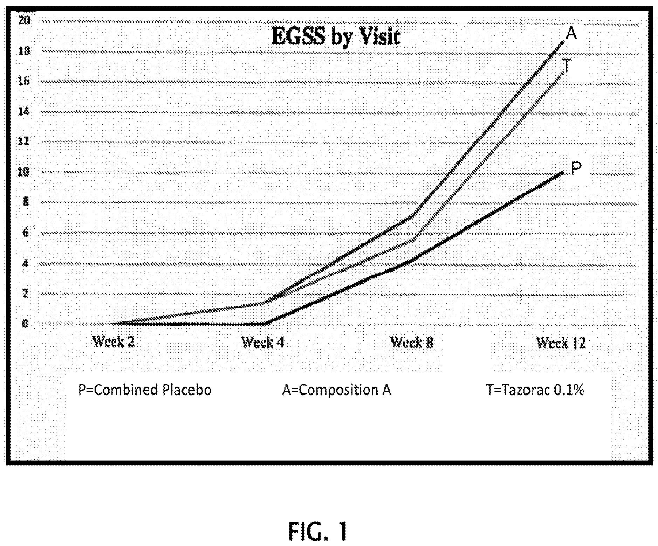

Figure 1 is a graph of EGSS by visit.
Figure 2 is a graph of change in inflammatory lesions from baseline.
Figure 3 is a graph of change in non-inflammatory lesions from baseline.
Figure 4 are graphs of cutaneous tolerability.
Figure 5 includes graphs of cutaneous safety.

### DETAILED DESCRIPTION OF THE INVENTION

In general, the present invention provides topical compositions for use in treating acne.

Throughout this disclosure, unless otherwise indicated, the concentration of an ingredient of the composition is in percent by weight of the total composition.

The topical pharmaceutical composition comprises tazarotene, or a pharmaceutically acceptable salt of tazarotenic acid, at a concentration below that which is presently utilized in topical formulations.

In the present invention, tazarotene or the tazarotenic acid salt is present in the composition in the range from about 0.01 to about 0.045 wt%, or from about 0.02 to about 0.045 wt%, or from about 0.03 to about 0.045 wt%, or at about 0.045 wt%. Specific concentrations of this component may be 0.01 wt%, 0.015 wt%, 0.02 wt%, 0.025 wt%, 0.03 wt% 0.035 wt%, 0.04 wt%, and 0.045 wt%.

The topical pharmaceutical composition comprises an oil-in-water emulsion as a carrier vehicle, in which an internal oil phase is dispersed in a continuous aqueous phase. The emulsion may be a macroemulsion, a microemulsion, or a nanoemulsion. The composition has the dosage form of a lotion.

In addition to the tazarotene active ingredient, the composition of the present invention may comprise one or more dermatologically acceptable excipients, such as liquid oils, waxes viscosity-modifying agents, thickening agents, gelling agents, alcohols, surfactants, chelating agents, buffers, preservatives, humectants, emollients, stabilizers, diluents, dispersing agents, emulsifiers, wetting agents, stabilizers, pH adjusters, solvents or cosolvents.

The composition of the invention may desirably contain a thickening agent to provide viscosity so that the formulation is provided in the form of a lotion. The thickening agent may be miscible or soluble in an aqueous fluid. Non-limiting examples of suitable thickening agents include acacia, alginic acid and its salts, hyaluronic acid and its salts, carbomers (also known as carboxy vinyl polymers, which are cross-linked polyacrylic acid), carboxymethylcellulose, ethylcellulose, gelatin, hydroxyethyl cellulose, hydroxypropyl cellulose, hydroxypropylmethyl cellulose, methylcellulose, poloxamers, polyvinylpyrrolidone, polyvinyl alcohol, tragacanth, xanthan gum, magnesium aluminum silicate, and bentonite. The thickening agent may also reside in the oil or lipophilic portion of the formulation. Examples of suitable lipophilic thickening agents include cetyl alcohol, stearyl alcohol, glyceryl stearate, white beeswax, microcrystalline wax, hydrogenated polyisobutane polymers, and emulsifying wax.

A suitable group of thickening agents is carbomers, such as Carbopol^{®} and polycarbophil (The Lubrizol Corporation, Wickliffe, Ohio). Carbopol^{®} homopolymers are polymers of acrylic acid crosslinked with allyl sucrose or allylpentaerythritol. Carbopol^{®} copolymers are polymers of acrylic acid and C₁₀-C₃₀ alkyl acrylate crosslinked with allylpentaerythritol. Carbopol^{®} interpolymers are carbomer homopolymers or copolymers that contain a block copolymer of polyethylene glycol and a long chain alkyl acid ester. Noveon^{®} polycarbophil is a polymer of acrylic acid crosslinked with divinyl glycol.

A surfactant or emulsifier is optionally included, if desired or required. Pharmaceutically acceptable anionic, cationic, or non-ionic surfactants may be included in a composition of the present invention. Non-ionic surfactants are preferred. Non-limiting examples of non-ionic surfactants are Octoxynol (also known as Macrogol tetramethylbutylphenyl ether, octylphenoxy polyethoxyethanol, or polyoxyethylene octylphenyl ether), such as Octoxynol 1, 3, 5, 8, 9, 10, 12, 13, 16, 30, 40, 70 (wherein the number indicates the number of repeating oxyethylene units), or other Octoxynols that comprise different numbers of repeating units of oxyethylene in the side chain, sorbitan esters (such as sorbitan monooleate and sorbitan monostearate, commonly known by their trade names Span 80 and Span 60), polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween^{®} 80, Tween^{®} 60, Tween^{®} 20), poloxamers (synthetic block polymers of ethylene oxide and propylene oxide, such as those commonly known by their trade names of Pluronic^{®}; e.g., Pluronic^{®} F127 or Pluronic^{®} F108), or poloxamines (synthetic block polymers of ethylene oxide and propylene oxide attached to ethylene diamine, such as those commonly known by their trade names of Tetronic^{®}; e.g., Tetronic^{®} 1508 or Tetronic^{®} 908, etc.), other nonionic surfactants such as Brij^{®} (polyoxyethylene alkyl ether having a formula of CH₃-(CH₂)₁₀₋₁₆-(O-C₂H₄)₁₋₂₅-OH), Myrj^{®} (stearic acid esterified with polyoxyethylene having 40-100 repeating oxyethylene units), and long chain fatty alcohols (e.g., oleyl alcohol, stearyl alcohol, myristyl alcohol, docosahexaenoyl alcohol, etc.) with carbon chains having about 12 or more carbon atoms (e.g., such as from about 12 to about 24 carbon atoms).

In addition, polymeric emulsifiers such as those known under the trade name Pemulen^{™} (The Lubrizol Corporation, Wickliffe, Ohio) may be used. These are polymers of acrylic acid, modified by long chain (C₁₀-C₃₀) alkyl acrylates, and crosslinked with allylpentaerythritol.

An anionic emulsifier may be used, such as sodium or potassium oleate, triethanolamine stearate, sodium lauryl sulfate, sodium dioctyl sulfosuccinate, and sodium docusate. Less preferred are cationic emulsifiers such as quaternary ammonium salts. Still other emulsifiers include glyceryl monostearate, polyoxyethylene monooleate, polyoxyethylene monostearate, polyoxyethylene monolaurate, potassium oleate, sodium lauryl sulfate, sodium oleate, sorbitan monolaurate, sorbitan monopalmitate, sorbitan monostearate, sorbitan tristearate, sorbitan monooleate, sorbitan trioleate, triethanolamine oleate, polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, and polyoxyethylene sorbitan trioleate.

The formulation desirably contains a dermatologically acceptable humectant such as glycerin, sorbitol, hexylene glycol, propylene glycol, or urea. In addition, the formulation may contain an emollient such as petrolatum, lanolin, mineral oil, light mineral oil, stearic acid, cyclomethicone, or dimethicone. Chelating agents such as EDTA and its salts may be included in a formulation of the present invention.

The liquid oil component of the composition includes one or more materials that are practically insoluble or insoluble in water and which are liquid at room temperature. For example, in one embodiment, the liquid oil component of the composition includes one or more materials that are practically insoluble or insoluble in water and which are liquid at room temperature of 22 °C. The liquid oil component may be selected from one or more ingredients from the group consisting of dicarboxylic acid esters ("DCAE"), monocarboxylic acid esters ("MCAE"), fish-liver oil, long-chain triglycerides (wherein each side chain has 14-18 carbons, such as peanut oil, sesame oil, coconut oil, sunflower oil, corn oil, olive oil, cotton seed oil, or derivatives thereof), propylene glycol diesters, medium-chain triglycerides (such as those wherein each side chains has 8-10 carbons; e.g., capric/caprylic acid triglycerides), hydrocarbons like mineral oil, light mineral oil, squalene, and squalane, fatty alcohols (such as octyldodecanol and isostearyl alcohol), and fatty acids (such as isostearic acid and oleic acid).

In some embodiments, the liquid oil component comprises a dicarboxylic acid ester and light mineral oil. In some other embodiments, the liquid oil component comprises one or more long-chain triglycerides.

The formulation may include other lipophilic liquids in an amount that is sufficient to be miscible with the dicarboxylic acid ester and/or monocarboxylic acid ester. The lipophilic liquid may be an emollient such as lanolin oil, mineral oil, light mineral oil, isostearic acid, squalene, octyldodecanol, fractionated coconut oil, cyclomethicone, or dimethicone.

In addition to the liquid oil component, the formulation may contain water insoluble or practically insoluble ingredients that are not liquid at room temperature, but are soluble in the liquid oil component.

A DCAE that is suitable for the present invention has the formula R₁OOC-(CH₂)ₙ-COOR₂, wherein R₁ and R₂ are alkyl groups containing between 1 and 4 carbons or aryl groups and may be the same or may be different and wherein (CH₂)ₙ is a straight or branched chain and n is between 1 and 12. Examples of DCAEs containing one or more aryl groups are dibenzyl esters of dicarboxylic acids. A preferred dicarboxylic acid ester is diethyl sebacate, which has the formula CH₃CH₂OOC-(CH₂)₈-COOCH₂CH₃. Examples of other suitable dicarboxylic acid esters (where R₁ and R₂ are the same) are dimethyl, diethyl, dipropyl, diisopropyl, dibutyl and diisobutyl esters such as oxalate, malate, succinate, glutarate, adipate, pimelate, suberate, and azalate. Examples of suitable dicarboxylic acid esters (where R₁ is different from R₂) are methyl ethyl, methyl propyl, methyl butyl, methyl isopropyl, ethyl propyl, ethyl butyl, ethyl isopropyl, and propyl butyl esters such as oxalate, malate, succinate, glutarate, adipate, pimelate, suberate, azalate, and sebacate.

In some aspects, diethyl sebacate is included at 0.1 to 20 wt%, or at 0.5 to 10 wt%, or at 2 to 4 wt% of the weight of the composition.

Alternatively, or in combination with the DCAE, the formulation may contain a MCAE. The MCAE that is suitable for the present invention has the formula CH₃-(CH₂)ₙ-COOR₁, wherein R₁ is an alkyl group containing between 1 and 4 carbons or an aryl group, and wherein (CH₂)ₙ is straight or branched chain and n is between 1 and 12. Examples of such monocarboxylic acid esters include methyl, ethyl, propyl, isopropyl, butyl, or an aryl such as benzyl formate, acetate, propionate, butyrate, valerate, laurate, myristate, palmitate, and stearate. Examples of preferred monocarboxylic acid esters are isopropyl palmitate and isopropyl myristate.

The liquid oil phase may beneficially be used to dissolve one or more of the active ingredients within the emulsion. In one embodiment the tazarotene component is dissolved in the liquid oil phase within the formulation at room temperature. In another embodiment the tazarotene component is suspended within the formulation at room temperature. In the case wherein the tazarotene component is suspended in the formulation, this suspended active ingredient may be micronized, namely that the mean particle size is preferably about 25 microns in diameter or less.

In one aspect, a composition of the present invention comprises the ingredients at the concentrations shown in Table 1.

**Table 1**

| Compositions of the Present Invention for Treating Skin Diseases | | | |
|---|---|---|---|
| Ingredient | Concentration (wt%) | | |
| | Range 1 | Range 2 | Range 3 |
| Tazarotene or Tazarotenic Acid Compound | 0.01-0.045 | 0.02-0.045 | 0.03-0.045 |
| Emollient, Solvent, and/or Thickener | 0.5-40 | 1-25 | 2-20 |
| Emulsifier | 0.25-10 | 0.5-7 | 1-5 |
| Humectant | 0-15 | 2-12 | 10 |
| Polymeric Thickener | 0.05-2 | 0.1-1.5 | 0.3-1 |
| Pharmaceutical Aids | q.s. | q.s. | q.s. |
| Purified water | q.s. to 100 | q.s. to 100 | q.s. to 100 |

Examples of compositions of the present invention are shown in Table 2.

**Table 2**

| Some Emulsion Compositions of the Present Invention for Treating Acne | | | | | |
|---|---|---|---|---|---|
| Ingredient | Function | Concentration (wt%) | | | |
| | | Range 1 | Range 2 | Range 3 | Composition A |
| Tazarotene | Retinoid | 0.02-0.045 | 0.03-0.045 | 0.04-0.045 | 0.045 |
| Diethyl sebacate | Liquid Oil & Solvent | 1-5 | 2-4 | 2.5-3.5 | 2.97 |
| Light mineral oil | Liquid Oil & CoSolvent | 5-15 | 5-10 | 7.5-8.5 | 8.03 |
| Sorbitan monooleate | Surfactant/ Emulsifying Agent | 0.01-1 | 0.02-0.5 | 0.05-0.2 | 0.1 |
| Sorbitol solution, 70% | Humectant | 5-15 | 7-12 | 10-11 | 10.7 |
| Methyl paraben | Antimicrobial Preservative⁽¹⁾ | 0.05-0.3 | 0.1-0.3 | 0.1-0.2 | 0.17 |
| Propyl paraben | Antimicrobial Preservative⁽¹⁾ | 0.01-0.1 | 0.01-0.05 | 0.02-0.04 | 0.03 |
| Edetate disodium dihydrate | Chelating Agent⁽¹⁾ | 0.02-0.1 | 0.02-0.7 | 0.03-0.06 | 0.05 |
| Carbomer copolymer type B (e.g., Pemulen^{™} TR-1) | Emulsifying Agent | 0.1-1 | 0.2-0.7 | 0.3-0.5 | 0.4 |
| Carbomer homopolymer type A (e.g., Carbomer 981) | Thickener | 0.2-1.5 | 0.3-1 | 0.5-0.7 | 0.6 |
| Sodium hydroxide, 10% Solution | pH-adjusting Agent(') | q.s. to pH of 5.5 ± 0.5 | q.s. to pH of 5.5 ± 0.5 | q.s. to pH of 5.5 ± 0.5 | q.s. to pH of 5.5 ± 0.5 |
| Purified water | Carrier | q.s. to 100 | q.s. to 100 | q.s. to 100 | q.s. to 100 |

| | | | | | |
|---|---|---|---|---|---|
| Note: ⁽¹⁾ These ingredients are broadly classified as Pharmaceutical Aids. | | | | | |

A lotion having a composition as shown Composition A of Table 2 may be prepared as follows.

A separate aqueous phase is made. In a manufacturing vessel equipped with a mixing implement (such as a propeller) and temperature control, purified water and disodium edetate dihydrate are combined and the mixture is agitated until a clear solution is achieved. Sorbitol, methylparaben, and propylparaben are then added to the mixture. The mixture is continuously mixed and heated to approximately 75 °C. The mixture is agitated until a solution is obtained. The mixture is then removed from the heat source and allowed to cool to below 40 °C with continued mixing. With continuous mixing, Carbopol^{®} 981 and Pemulen^{™} TR-1 carbomers are added to the mixture and dispersed. Mixing continues until the two carbomers are fully dispersed and hydrated.

A separate oil phase is made. In a vessel equipped with a mixing implement such as a propeller, diethyl sebacate, and tazarotene are combined. The mixture is agitated until a solution is achieved. With continuous mixing, light mineral oil and sorbitan monooleate are added. Mixing is continued until a solution is obtained.

In a separate vessel, an approximate 2.5N solution of sodium hydroxide is prepared.

With high speed mixing, the oil phase containing the active ingredient (tazarotene) is added to the aqueous phase. Mixing is continued until a homogeneous emulsion is obtained. Mixing speed is decreased and mixing continued for an additional time of 10 minutes to 1 hour. With continuous mixing, an appropriate amount of the sodium hydroxide solution is added incrementally to obtain a pH of 5.5 ± 0.5. Mixing continues further until a homogeneous lotion is obtained, such as for 30 minutes to 3 hours.

A clinical study in acne patients was conducted to compare the efficacy of a composition of the present invention containing tazarotene (the "Composition A" lotion of Table 2).

A first placebo ("Lotion Placebo") corresponded to and had a similar viscosity as Composition A but lacked tazarotene. A second placebo ("Cream Placebo") corresponded to the Lotion Placebo but employed carbomer homopolymer type C in place of carbomer homopolymer type A to yield a higher viscosity emulsion with a viscosity similar to 0.1% tazarotene cream employed in the study. This allows for better blinding of the test formulations and control of the clinical study. In the following results, data for Lotion Placebo and Cream Placebo may be combined and reported as "Combined Placebo".

Additionally, commercially available 0.1% tazarotene cream (Tazorac^{®} 0.1% cream) was employed in this clinical study for comparison purposes.

A double blind, multi-site, randomized clinical study was conducted on patients suffering from acne vulgaris, whereby neither the acne patient nor the investigator knew the identity of the test composition assigned. 210 patients were randomized to receive either Composition A lotion, Tazorac^{®} 0.1% cream, Placebo Lotion and Placebo Cream at a 2:2:1:1 randomization:
69 subjects "Composition A lotion"
72 subjects "Tazorac^{®} 0.1% cream"
34 subjects "Lotion Placebo"
35 subjects "Cream Placebo".

Primary inclusion criteria were moderate to severe acne, inflammatory lesion count of at least 20 but no more than 40, and non-inflammatory lesion count of at least 20 but no more than 100. The blindly labeled lotions were applied to the affected area once daily for twelve weeks, with assessments at 2 weeks, 4 weeks, 8 weeks and 12 weeks.

The investigator monitored the efficacy at each study visit by assessing the treatable area, determining the Evaluator's Global Severity Score (EGSS), and determining the grade of improvement. EGSS included the following grades:
Clear = 0; Almost Clear = 1; Mild = 2; Moderate = 3; Severe = 4.

Clinical Efficacy was determined primarily based on the percentage of subjects who were treatment successes at 12 weeks. To be judged as a treatment success, as reported in Tables 4, 6, and 7, subjects had to show at least two-grade improvement from the baseline and EGSS score equating to "clear" or "almost clear". Also, the number of inflammatory and non-inflammatory lesions were assessed.

Table 3 reports Baseline Characteristics.

**Table 3**

| **Baseline Characteristics - Inflammatory Lesions and Non-inflammatory lesions** | | | | |
|---|---|---|---|---|
| | | Combined Placebo N=69 | Composition A N=69 | Tazorac^{®} 0.1% Cream N=72 |
| Inflammatory Lesions | | | | |
| | Mean (SD) | 27.2 (5.49) | 28.3 (6.0) | 27.3 (5.95) |
| | Median | 26.0 | 27.0 | 26.0 |
| Non-inflammatory Lesions | | | | |
| | Mean (SD) | 36.6 (13.17) | 37.6 (14.70) | 36.6 (13.31) |
| | Median | 34.0 | 34.0 | 34.0 |

Table 4 reports treatment % success on an intention-to-treat (ITT) basis.

**Table 4**

| **EGSS (Clear or Almost Clear) by Visit (ITT)** | | | |
|---|---|---|---|
| | Combined Placebo N=69 | Composition A N=69 | Tazorac^{®} 0.1% Cream N=72 |
| Week 2 | 0% | 0% | 0% |
| Week 4 | 0% | 1.4% | 1.4% |
| Week 8 | 4.3% | 7.2% | 5.6% |
| Week 12 | 10.1% | 18.8% | 16.7% |

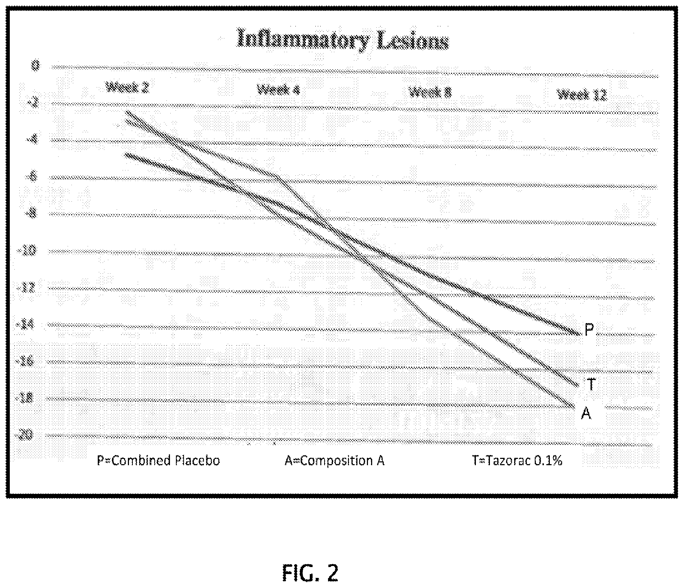

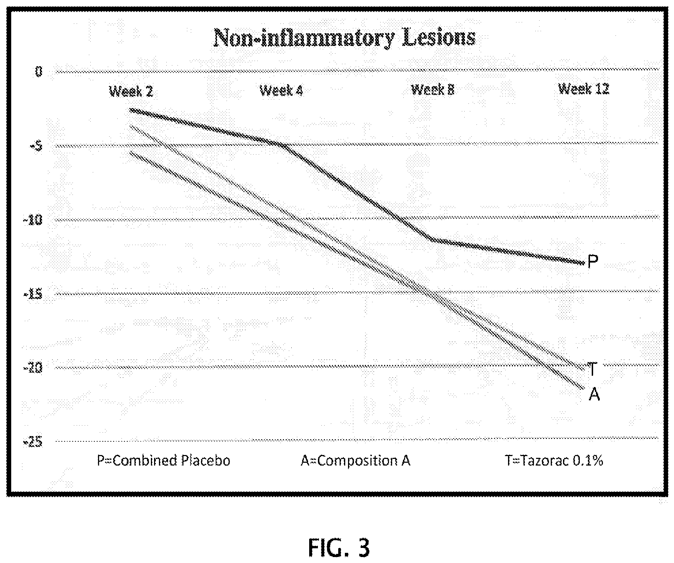

Table 5 reports absolute change in inflammatory lesions from baseline. Change in inflammatory and non-inflammatory lesions from baseline is reported in Figures 2 and 3.

**Table 5**

| **Inflammatory Lesions by Visit Absolute Change from Baseline** | | | |
|---|---|---|---|
| | Combined Placebo N=69 | Composition A N=69 | Tazorac^{®} 0.1% Cream N=72 |
| Week 2 | -4.7 | -2.9 | -2.4 |
| Week 4 | -7.2 | -5.8 | -7.8 |
| Week 8 | -10.9 | -13.2 | -12.0 |
| Week 12 | -14.0 | -18.1 | -16.8 |

Table 6 reports treatment % success on an intention-to-treat basis for EGSS. For lesions, absolute change from baseline to Week 12 in mean inflammatory and non-inflammatory counts is reported.

**Table 6**

| **Treatment % Success (ITT) at 12 Weeks** | | | | |
|---|---|---|---|---|
| | | Combined Placebo N=69 | Composition A N=69 | Tazorac^{®} 0.1% Cream N=72 |
| EGSS - Clear or Almost Clear | | 10.1% | 18.8% | 16.7% |
| Inflammatory Lesions | | | | |
| | Absolute Change | -14.0 | -18.1 | -16.8 |
| | % Change | -51.41% | -63.75% | -59.99% |
| Non-inflammatory Lesions | | | | |
| Absolute Change | | -13.1 | -21.6 | -20.3 |
| | % Change | -35.18% | -56.86% | -54.09% |

Table 7 reports treatment % success on a per-protocol basis for EGSS. For lesions, absolute change from baseline to Week 12 in mean inflammatory and non-inflammatory counts is reported.

**Table 7**

| **Treatment % Success (PP)** | | | |
|---|---|---|---|
| | Combined Placebo N=57 | Composition A N=54 | Tazorac^{®} 0.1% Cream N=56 |
| EGSS - Clear or Almost Clear | 12.3% | 20.4% | 16.1% |
| Inflammatory Lesions | -14.2 | -18.3 | -18.2 |
| Non-inflammatory Lesions | -11.8 | -20.8 | -21.9 |

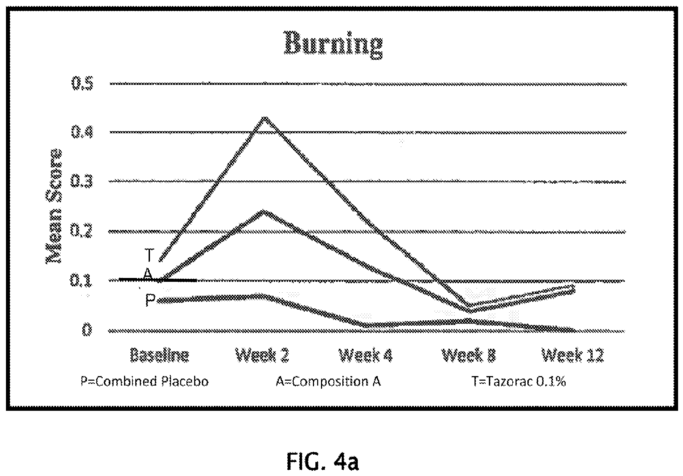

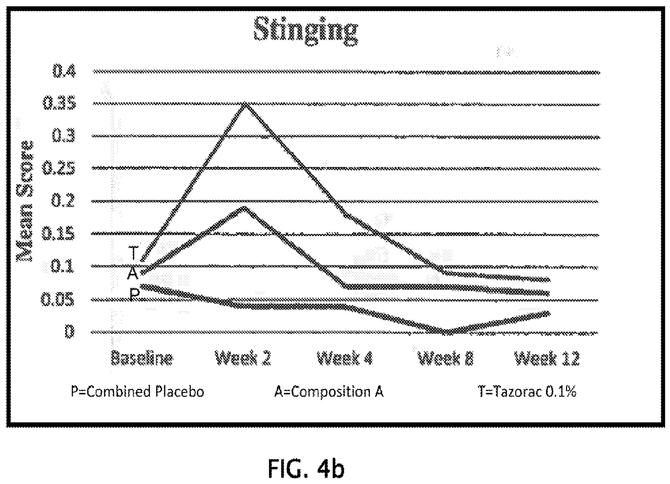

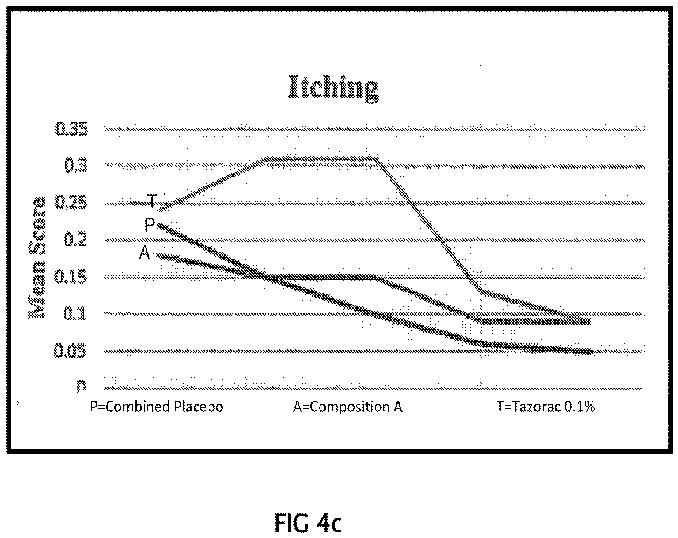

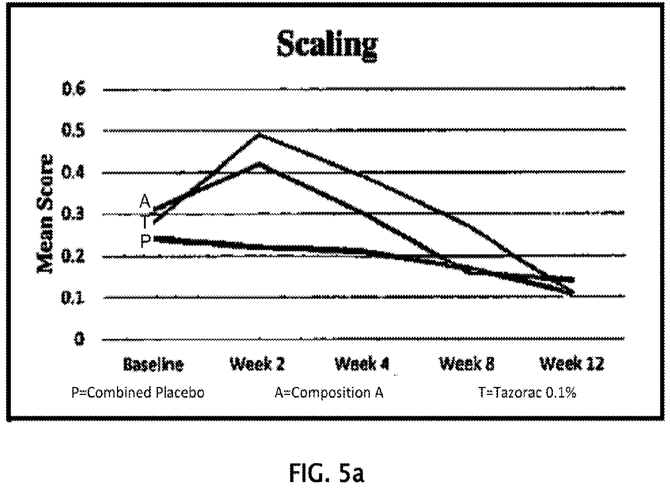

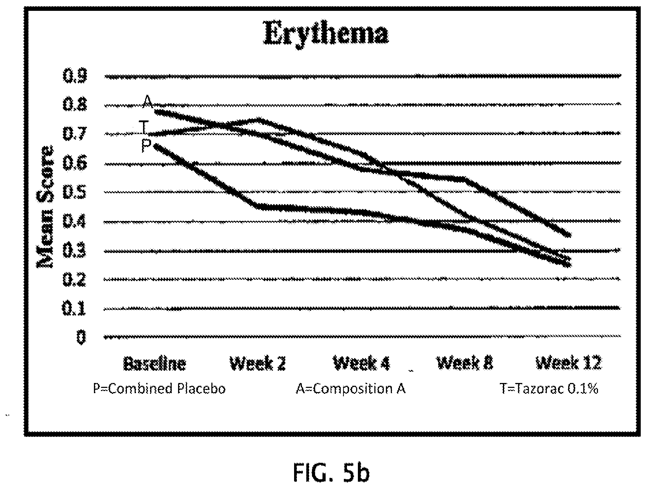

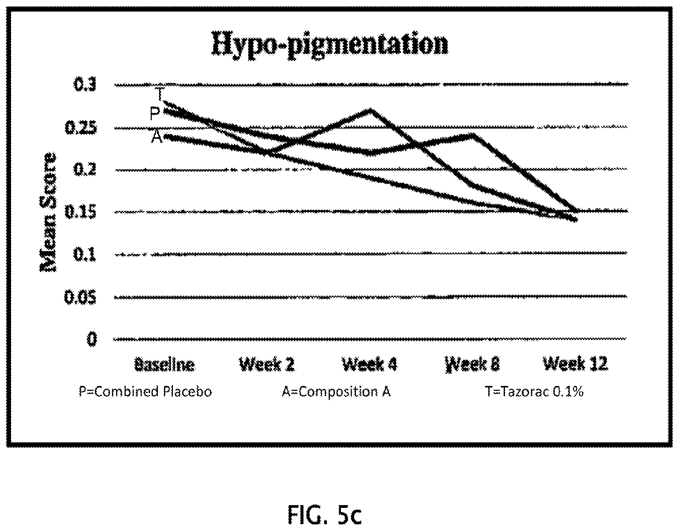

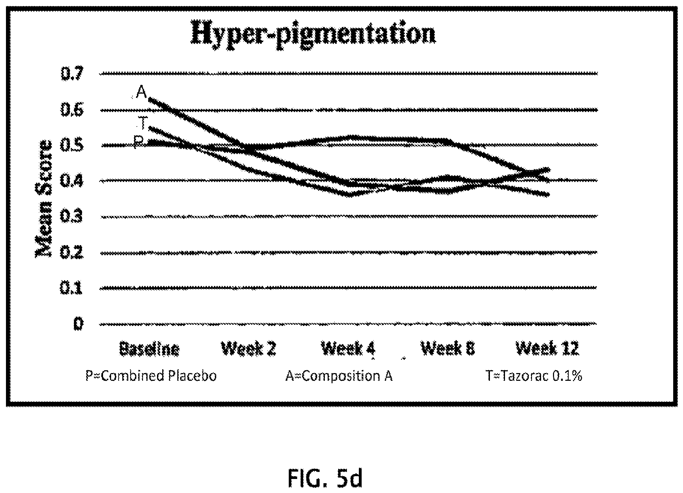

The compositions were tested for cutaneous safety and tolerability. Cutaneous tolerability was assessed by burning, stinging or itching reported by the subjects. This data is summarized in Figure 4. Cutaneous safety was assessed by scaling, erythema, hypo-pigmentation and hyper-pigmentations as evaluated by the investigator. This data is summarized in Figure 5.

Table 8 reports treatment emergent adverse event characteristics.

**Table 8**

| **Treatment Emergent Adverse Event Characteristics** | | | |
|---|---|---|---|
| | Combined Placebo | Composition A | Tazorac^{®} 0.1% Cream |
| % Subjects Reporting Any Adverse Event | 13.4% | 14.7% | 26.8% |
| % Subjects Reporting Any Serious Adverse Event | 0% | 0% | 0% |
| % Subjects Discontinued Study Due to Adverse Event | 0% | 0% | 1.4% |

Table 9 reports treatment % success for primary efficacy at week 12. For EGSS, to be judged as a treatment success, subjects had to show at least two-grade improvement from the baseline and EGSS score equating to "clear" or "almost clear". For inflammatory and non-inflammatory lesions, absolute change from baseline to week in mean inflammatory and non-inflammatory counts is reported.

Notably, this clinical study showed that Composition A, containing less than half of the tazarotene active ingredient, had a numerically better efficacy than the Tazorac^{®} 0.1% Cream. Also, the adverse event profile was lower for Composition A than for Tazorac^{®} 0.1% Cream.

Described herein is the treatment of acne. The treatment comprises topically applying to an affected area of the body of a subject suffering from acne any one of the compositions of the present invention, as disclosed herein, one or more times per day for a period of time sufficient to treat such acne. For example, such a period of time may be 1 to 30 days or longer as needed For example, such a period of time may be one week, two weeks, four weeks, eight weeks, twelve weeks, or longer as needed. For example, a composition of the present invention is applied topically to affected areas of the body once per day for 7-14 days. Alternatively, it may be applied two or three times per day for 7-14 days. Alternatively, it may be applied once per day for one week to six months. For example, it may be applied once per day for two weeks, four weeks, eight weeks, or twelve weeks. The treatment may be stopped for 1-7 days (e.g., 2, 3, 4, 5, 6, or 7 days) after an extended treatment period before it is resumed for another extended treatment period. Such an extended period may be 7 days, 7-14 days, 7-21 days, 7-30 days, or longer before more treatment is needed or desired.

Described herein is the treatment of acne topically with any of the pharmaceutical compositions of this invention, wherein the composition is applied once daily for more than 2 weeks, such as 4 weeks, for 6 weeks, for 8 weeks or for 12 weeks, without any serious adverse events, and/or with an improved adverse event profile compared to 0.1% tazarotene cream.

Described herein is the treatment of acne topically with any of the pharmaceutical compositions of this invention, wherein the composition is applied once daily for 12 weeks, and has better clinical efficacy and a lower adverse event profile as compared to commercial compositions containing 0.1% tazarotene.

## Claims

1. A topical pharmaceutical composition for use in the treatment of acne, comprising:
tazarotene or a pharmaceutically acceptable tazarotenic acid salt present in the composition at 0.01 to 0.045 percent by weight of the composition; and
a dermatologically acceptable oil-in-water emulsion vehicle,
the topical pharmaceutical composition containing the tazarotene or the tazarotenic acid salt as a sole active pharmaceutical ingredient, wherein the topical pharmaceutical composition is a lotion.

2. The topical pharmaceutical composition for the use of claim 1, comprising tazarotene at 0.045 percent by weight of the composition.

3. The topical pharmaceutical composition for the use of any one of claims 1-2, wherein the tazarotene or tazarotenic acid salt is dissolved in a liquid oil component of the emulsion.

4. The topical pharmaceutical composition for the use of any one of claims 1-3, wherein an oil phase of the emulsion comprises a liquid oil component comprising a dicarboxylic acid ester (DCAE), a monocarboxylic acid ester (MCAE), or combinations thereof.

5. The topical pharmaceutical composition for the use of any one of claims 1-3, wherein an oil phase of the emulsion comprises diethyl sebacate.

6. The topical pharmaceutical composition for the use of any one of claims 1-5, wherein an aqueous phase of the emulsion comprises water and a carbomer homopolymer.

7. The topical pharmaceutical composition for the use of any one of claims 1-3, comprising:
an aqueous phase comprising water, a carbomer homopolymer, and a polymeric emulsifier;
an oil phase comprising at least one member selected from the group consisting of a dicarboxylic acid ester and a mineral oil, and
the tazarotene or the tazarotenic acid salt.

8. The topical pharmaceutical composition for the use of any one of claims 1-7, having a pH of 4 to 6.

9. The topical pharmaceutical composition for the use of any one of claims 1-8, wherein the pharmaceutical composition is topically applied to an affected area of a body of a subject suffering from acne.

10. The topical pharmaceutical composition for the use of claim 9, wherein said applying is carried out once per day for at least eight weeks, and optionally for at least twelve weeks.

11. The topical pharmaceutical composition for the use of claim 9 or 10, wherein said applying is to an affected area of a body of a subject suffering from acne vulgaris.

## Patentansprüche

1. Topische pharmazeutische Zusammensetzung zur Verwendung in der Behandlung von Akne, umfassend:
Tazaroten oder ein pharmazeutisch akzeptables Tazarotensäuresalz, das in der Zusammensetzung zu 0,01 bis 0,045 Gewichtsprozent der Zusammensetzung vorhanden ist; und
ein dermatologisch akzeptables Öl-in-Wasser-Emulsionsvehikel,
wobei die topische pharmazeutische Zusammensetzung das Tazaroten oder das Tazaroten-Salz als einzigen pharmazeutisch aktiven Wirkstoff enthält,
wobei die topische pharmazeutische Zusammensetzung eine Lotion ist.

2. Die topische pharmazeutische Zusammensetzung für die Verwendung nach Anspruch 1, umfassend Tazaroten in einer Menge von 0,045 Gewichtsprozent der Zusammensetzung.

3. Die topische pharmazeutische Zusammensetzung für die Verwendung nach einem der Ansprüche 1-2, wobei das Tazaroten oder Tazarotensäuresalz in einer flüssigen Ölkomponente der Emulsion gelöst ist.

4. Die topische pharmazeutische Zusammensetzung für die Verwendung nach einem der Ansprüche 1-3, wobei eine Ölphase der Emulsion eine flüssige Ölkomponente umfasst, umfassend einen Dicarbonsäureester (DCAE), einen Monocarbonsäureester (MCAE) oder Kombinationen davon.

5. Die topische pharmazeutische Zusammensetzung für die Verwendung nach einem der Ansprüche 1-3, wobei eine Ölphase der Emulsion Diethylsebacat umfasst.

6. Die topische pharmazeutische Zusammensetzung für die Verwendung nach einem der Ansprüche 1-5, wobei eine wässrige Phase der Emulsion Wasser und ein Carbomer-Homopolymer umfasst.

7. Die topische pharmazeutische Zusammensetzung für die Verwendung nach einem der Ansprüche 1-3, umfassend:
eine wässrige Phase, die Wasser, ein Carbomer-Homopolymer und einen polymeren Emulgator umfasst;
eine Ölphase, die mindestens ein Mitglied ausgewählt aus der Gruppe bestehend aus einem Dicarbonsäureester und einem Mineralöl umfasst, und
das Tazaroten oder das Tazarotensäuresalz.

8. Die topische pharmazeutische Zusammensetzung für die Verwendung nach einem der Ansprüche 1-7, aufweisend einen pH-Wert von 4 bis 6.

9. Die topische pharmazeutische Zusammensetzung für die Verwendung nach einem der Ansprüche 1-8, wobei die pharmazeutische Zusammensetzung topisch auf einen betroffenen Bereich eines Körpers eines an Akne leidenden Subjekts aufgetragen wird.

10. Die topische pharmazeutische Zusammensetzung für die Verwendung nach Anspruch 9, wobei besagtes Auftragen einmal täglich für mindestens acht Wochen und optional für mindestens zwölf Wochen durchgeführt wird.

11. Die topische pharmazeutische Zusammensetzung für die Verwendung nach Anspruch 9 oder 10, wobei besagtes Auftragen auf einen betroffenen Bereich eines Körpers eines an Acne vulgaris leidenden Subjekts erfolgt.

## Revendications

1. Composition pharmaceutique topique destinée à être utilisée dans le traitement de l'acné, comprenant :
du tazarotène ou un sel d'acide tazaroténique pharmaceutiquement acceptable présent dans la composition à raison de 0,01 à 0,045 % en poids de la composition ; et
un véhicule sous forme d'émulsion huile dans eau dermatologiquement acceptable,
la composition pharmaceutique topique contenant le tazarotène ou le sel d'acide tazaroténique comme seul ingrédient pharmaceutique actif,
dans laquelle la composition pharmaceutique topique est une lotion.

2. Composition pharmaceutique topique selon la revendication 1, comprenant du tazarotène à raison de 0,045 % en poids de la composition.

3. Composition pharmaceutique topique destinée à l'usage selon l'une quelconque des revendications 1 à 2, dans laquelle le tazarotène ou le sel d'acide tazaroténique est dissous dans un composant huileux liquide de l'émulsion.

4. Composition pharmaceutique topique destinée à être utilisée selon l'une quelconque des revendications 1 à 3, dans laquelle une phase huileuse de l'émulsion comprend un composant huileux liquide comprenant un ester d'acide dicarboxylique (DCAE), un ester d'acide monocarboxylique (MCAE) ou des combinaisons de ceux-ci.

5. Composition pharmaceutique topique destinée à être utilisée selon l'une quelconque des revendications 1 à 3, dans laquelle une phase huileuse de l'émulsion comprend du sébacate de diéthyle.

6. Composition pharmaceutique topique destinée à être utilisée selon l'une quelconque des revendications 1 à 5, dans laquelle une phase aqueuse de l'émulsion comprend de l'eau et un homopolymère de carbomère.

7. Composition pharmaceutique topique destinée à être utilisée selon l'une quelconque des revendications 1 à 3, comprenant :
une phase aqueuse comprenant de l'eau, un homopolymère de carbomère et un émulsifiant polymère ;
une phase huileuse comprenant au moins un élément choisi dans le groupe constitué par un ester d'acide dicarboxylique et une huile minérale, et
le tazarotène ou le sel d'acide tazaroténique.

8. Composition pharmaceutique topique destinée à être utilisée dans l'une quelconque des revendications 1 à 7, ayant un pH compris entre 4 et 6.

9. Composition pharmaceutique topique destinée à être utilisée selon l'une quelconque des revendications 1 à 8, dans laquelle la composition pharmaceutique est appliquée par voie topique sur une zone affectée du corps d'un sujet souffrant d'acné.

10. Composition pharmaceutique topique destinée à être utilisée selon la revendication 9, dans laquelle ladite application est effectuée une fois par jour pendant au moins huit semaines, et éventuellement pendant au moins douze semaines.

11. Composition pharmaceutique topique destinée à être utilisée selon la revendication 9 ou la revendication 10, dans laquelle ladite application est effectuée sur une zone affectée du corps d'un sujet souffrant d'acné vulgaire.
